# EUROPEAN PATENT APPLICATION

(11) **EP 4 706 586 A1**
(43) Date of publication of application: **11.03.2026**
(21) Application number: 24800204.0
(22) Date of filing: 26.04.2024
(51) Int. Cl.: A61C 7/00, A61C 9/00, A61C 19/04, A61B 5/00, G06T 17/00, G16H 30/00, G16H 50/50, B33Y 80/00, A61B 18/20

(54) **METHOD FOR MANUFACTURING ORTHODONTIC DEVICE BY USING INSERTION PATH**

(30) Priority: 04.05.2023 KR 20230058191
(71) Applicant: ODS Co. Ltd., Incheon 21990 (KR)
(72) Inventor: SIM, Miyoung, Incheon 21986 (KR); PARK, Junbeom, Incheon 21974 (KR)
(74) Representative: You Patent
(86) International application number: PCT/KR2024/005657
(87) International publication number: WO 2024/228520

(57) **Abstract**

The present invention relates to a program for designing an orthodontic appliance. The method comprises: displaying, on a screen, the insertion path for determining the insertion direction of the appliance (S01); designating a tooth targeted for the insertion path setting (S02); setting the insertion path for the designated tooth in an arbitrary direction (S03); displaying, on the screen, undercuts of each tooth caused by the set insertion path (S04); calculating and storing depth values of the undercuts for each tooth (S05); and calculating and storing the total sum of undercut volumes for each tooth according to the insertion path (S06).

## Description

### Technical Field

The present invention relates to a method (program) for designing an orthodontic appliance using an electronic information processing device such as a computer.

More specifically, it relates to a manufacturing method for producing an orthodontic appliance optimized for a patient's oral structure, wherein the oral structure (e.g., dentition information) is obtained by means of a 3D scanner and an orthodontic appliance is fabricated accordingly.

### Background Art

Conventionally, orthodontic appliances are manufactured as follows.

A practitioner first examines the patient's oral structure and produces a dental model identical in shape to the patient's teeth. Typically, the model is created by taking an impression of the oral structure and casting plaster. Using such a plaster model, a sheet-type polyol material is thermo-pressed to manufacture a transparent aligner.

More recently, methods employing 3D scanners have been adopted, wherein the patient's oral structure is scanned and stored, and the data is used to fabricate a dental model. Compared with impression-taking methods, this approach is more advanced.

Further, a method of directly fabricating transparent aligners using a 3D printer has also been introduced. Unlike conventional approaches where a dental model is first created, this method enables direct printing of the orthodontic appliance, eliminating the need for a model.

The present invention relates to a program for designing orthodontic appliances on a computer, which are directly produced by a 3D printer.

### Specific Instruction for Implementing the Invention

### Technical Problem

The invention addresses the challenge of manufacturing removable transparent orthodontic appliances by 3D printing,
wherein an insertion path corresponding to the insertion direction of the appliance is ideally determined to optimize fitting to the patient's dentition.

### Technical Solution

The invention provides a method comprising: displaying, on a screen, the insertion path for determining the insertion direction of the orthodontic appliance (S01); designating a tooth targeted for the insertion path setting (S02); and displaying, on the screen, the undercuts of each tooth caused by the set insertion path (S04).

### Effects of the Invention

The present invention provides an orthodontic appliance optimized for a patient's dentition by ideally setting an insertion path corresponding to the insertion direction of the appliance.

As a result, the orthodontic effect is maximized, thereby enhancing the therapeutic outcome while also facilitating attachment and detachment of the appliance to improve user convenience.

Furthermore, in designing the orthodontic appliance on a computer, the invention enables easy identification of the most ideal insertion path, thereby reducing the time and effort required for appliance manufacture.

In addition, by allowing the optimal insertion path to be readily set, the invention is expected to significantly improve the quality of orthodontic appliances even when operated by an inexperienced user.

### Brief Description of Drawings

Fig. 1: Flowchart of the manufacturing method.
Fig. 2: Screen showing insertion path and corresponding undercuts.
Fig. 3: Menu functions according to an embodiment.
Fig. 4: State after block-out (removal of undercuts).

### Best Mode for Implementing the Invention

The invention comprises the following steps:
displaying, on a screen, an insertion path for determining the insertion direction of the orthodontic appliance (S01); designating a tooth targeted for the insertion path setting (S02); arbitrarily setting the insertion path for the designated tooth (S03); displaying, on the screen, undercuts of each tooth caused by the set insertion path (S04); calculating and storing depth values of the undercuts for each tooth according to the insertion path (S05); calculating and storing the total sum of the undercut volumes for each tooth (S06); setting the insertion path in a direction different from the previously set one and repeating steps S04-S06 (S07); and selecting and storing the insertion path having the minimum total undercut volume obtained from step S07 (S08).

### Mode for Implementing the Invention

Hereinafter, a detailed description will be given of the method for manufacturing a transparent orthodontic appliance according to the present invention, step by step.

The technical terms used in the present specification are employed solely for the purpose of explaining the embodiments, and it is not intended to limit the scope of the invention by such terms. Unless specifically defined otherwise, the terms are to be understood as having the meanings commonly used and understood by those skilled in the art.

The present invention relates to a method (program) for designing a transparent orthodontic appliance using a computer. In order to implement the invention, a prior step of three-dimensional (3D) scanning of the patient's dentition is required. By using a 3D scanner, the inside of the oral cavity is carefully scanned to acquire image data of the dental structure, and the dental structure is displayed based on such information.

When the image of the patient's dentition is completely displayed, an orthodontic appliance optimized therefor is designed by means of the present invention using a computer.

The orthodontic appliance herein is intended for orthodontic correction of dentition (including horizontal movement, rotation, extrusion, and intrusion of teeth, i.e., all positional changes of the teeth), and is output by means of a 3D printer.

Although it is preferable that the orthodontic appliance according to the present invention be applied to a transparent aligner that covers the entire dentition (dental model), it is also applicable to an appliance that partially covers only a portion of the dentition (for example, an appliance covering only several adjacent teeth centered around the target tooth to be corrected).

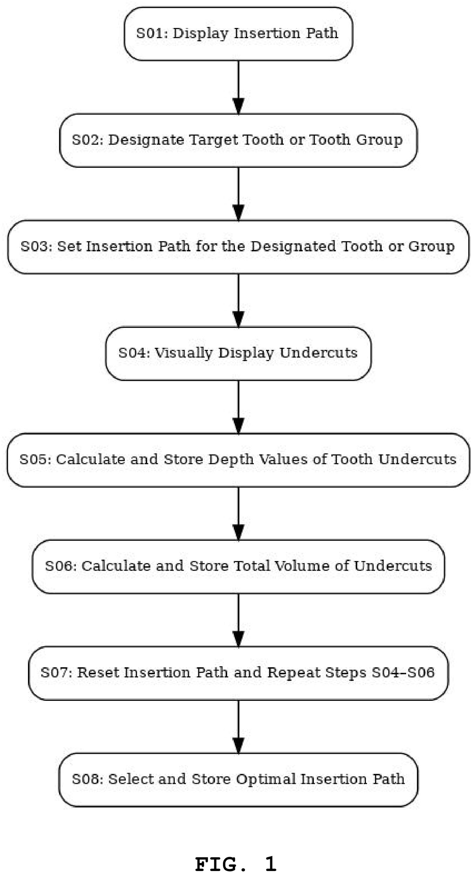

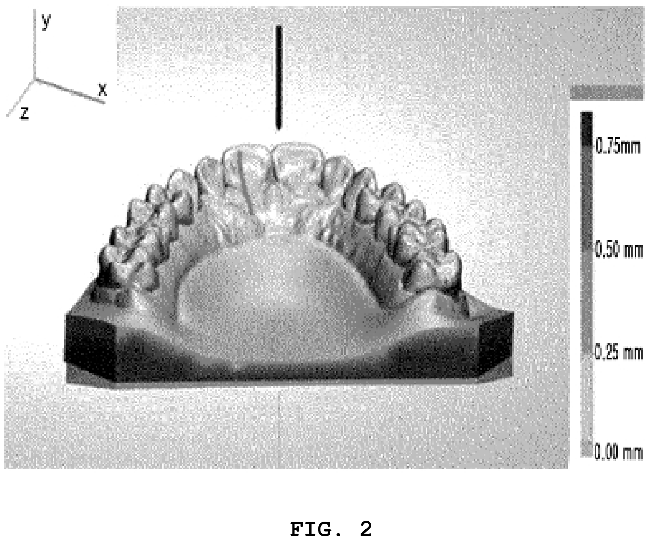

Figure 1 is a flowchart illustrating the method for manufacturing an orthodontic appliance according to the present invention, and Figure 2 shows a screen displaying an insertion path and the undercuts corresponding thereto.

In step S01, the insertion path for determining the insertion direction of the orthodontic appliance is displayed on the screen. In step S02, a tooth is designated as the target for insertion path setting.

The problem of setting the insertion path arises because the axial orientation of each tooth differs slightly. To achieve excellent orthodontic performance, the appliance must closely adhere to the dentition and be supported by the teeth. Thus, the manner in which the insertion path is set directly affects the appliance's performance.

The insertion path refers to the direction in which the appliance is worn onto the teeth. Although the vertical axis (Y-axis in the drawing) serves as a basic reference, the path should be set at an inclination that ensures both effective orthodontic force and ease of insertion and removal, since the appliance is removable rather than fixed.

As illustrated in Figure 2, when an arbitrary insertion path is set, the undercuts corresponding to that path are displayed for each tooth. The present invention is characterized in that the operator designates a certain range of teeth and determines the optimal insertion path for the designated group; however, Figure 2 shows an example where undercuts for the entire dentition are displayed in accordance with the set insertion path.

The insertion path can be set by entering arbitrary angular values inclined from the Y-axis toward the X- and/or Z-axis. For example, if +3° is input for the X-axis and -5° for the Z-axis, the insertion path will be displayed as inclined 3° in the positive X direction and 5° in the negative Z direction.

Alternatively, the insertion path may be set interactively by moving a cursor near the insertion line, thereby activating a tilt function, and adjusting the path in the X- and/or Z-axis direction as desired.

In step S02, the operator designates the teeth subject to the insertion path setting. The designated teeth may be a single tooth or, alternatively, multiple adjacent teeth grouped together.

In step S03, the insertion path is arbitrarily set for the designated tooth (or group of teeth). In step S04, the undercuts of each tooth resulting from the set insertion path are displayed on the screen. In step S05, the depth values of the undercuts for each tooth according to the insertion path are calculated and stored.

When an insertion path is arbitrarily set, the undercuts appear accordingly.

Teeth generally vary in transverse diameter along their height, with the greatest diameter occurring at the height of contour (the bulging portion). Beyond this portion toward the gingiva, the diameter decreases, forming undercuts. Undercuts may naturally occur in normal teeth, and may be more pronounced in cases of trauma or dental caries.

Although Figure 2 illustrates an example for the mandibular dentition, in the case of the maxilla, the undercuts are located at the upper portions of the teeth. The principle is the same, and a detailed description thereof is omitted.

Depending on how the insertion path is set, the shape of each undercut varies, since each tooth has a different inclination and undercut geometry.

When one insertion path is set, the undercuts of the designated teeth are displayed visually, as shown in Figure 2 (step S04).

Although Figure 2 shows all undercuts of the dentition, it is also possible to display only those of the designated teeth, depending on the operator's preference.

Each tooth undercut is displayed in a color different from that of the tooth for easy recognition, and is further color-coded according to depth values.

Here, the depth refers to the horizontal dimension of the undercut (e.g., X- or Y-axis direction in Figure 2). Shallower undercuts appear yellow, while deeper ones appear red.

Thus, the undercut depth values are continuously calculated and stored for each tooth.

The operator can thereby instantly recognize the presence and magnitude of undercuts corresponding to a given insertion path (step S05).

In step S06, the total volume of undercuts for each tooth corresponding to the set insertion path is calculated and stored. Once the undercuts for a specific insertion path are determined, the computer calculates the volume of each tooth's undercut, sums them, and stores the total value. The operator may check the cumulative volume at any desired time.

In step S07, the insertion path is reset to a different direction, and steps S04 through S06 are repeated.

By changing the inclination, the operator can observe how the shape, depth, and volume of the undercuts vary. Step S07 may be performed once or multiple times until an optimal insertion path is determined, either manually or automatically.

When the inclination of the insertion path changes, the undercuts of each tooth vary in shape, depth, and volume. Since each tooth has a different vertical inclination and unique morphology, a change in the inclination of the insertion path results in variations in the shape and volume of the undercuts for each tooth.

In Step S07, the user sets the insertion path differently from the previous setting and, at each adjustment, is able to check the shape of the undercuts for each tooth as well as the total sum of the volumes of the undercuts. Step S07 may be performed only once; however, depending on the operator's choice, it may also be performed three or more times until the optimal insertion path is determined, and may be carried out any other number of times as needed.

In certain cases, where a particular tooth is severely inclined in one direction, the undercut volume may become exaggerated in that direction, distorting the overall calculation. In such cases, it is preferable to exclude the undercut of the severely inclined tooth from the volume calculation.

Step S08 is a step of selecting and storing the insertion path having the minimum value among the total volume values of the undercuts obtained in Step S07.

In order for the orthodontic appliance to effectively exert orthodontic force on the teeth, it is required to be closely attached to the teeth. For the close attachment of the orthodontic appliance, an appliance capable of minimizing the degree of undercut is required. On the other hand, if only the securing of orthodontic force is pursued in the production of the appliance, the removability (ease of use) of the appliance may be significantly reduced. Therefore, it is required to establish an insertion path that can secure removability while maximizing orthodontic force.

The present invention proposes, as the most ideal insertion path, an insertion path in which the sum of the volumes of the undercuts calculated for each tooth is minimized.

The method for designing an orthodontic appliance according to the present invention aims to provide an orthodontic appliance optimized for the target teeth, which can be easily attached and detached while ensuring the maximum orthodontic force.

According to the present invention, even an unskilled person can easily identify the most ideal insertion path, and it is therefore expected that the fabrication of an optimized orthodontic appliance will be possible.

On the other hand, the present invention is also intended to provide an orthodontic appliance which, while ensuring wearability, can exert excellent orthodontic force by setting a plurality of insertion paths. The practitioner may designate a first group of teeth to which one insertion path is applicable, and execute Steps S04 to S06 to find the optimal insertion path for the designated first group. Thereafter, other teeth may be designated as a second group, and likewise Steps S04 to S06 may be executed to find the optimal insertion path for the second group (S021, S031).

This process may further be extended to a third group, a fourth group, and so on.

Since the orthodontic appliance according to the present invention has a certain degree of elasticity, wearability can be ensured even when the appliance partially has different insertion paths. Furthermore, by setting the insertion path differently for each group of teeth, it is expected that optimal orthodontic force tailored to the characteristics of each tooth will be applied to the teeth to be corrected.

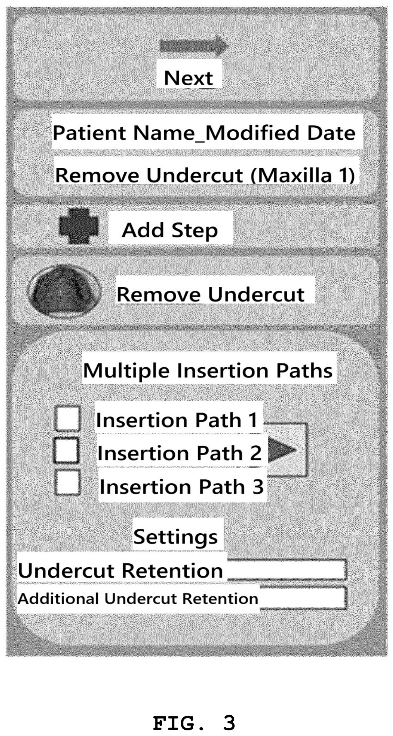

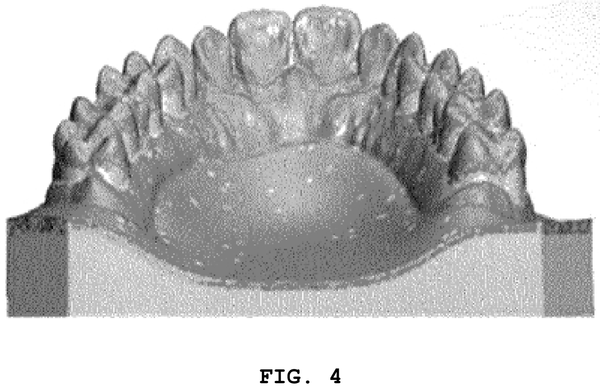

Figure 3 illustrates a menu for performing a specific function according to an embodiment of the invention, and Figure 4 shows a state in which undercuts have been removed in accordance with the execution of the menu in Figure 3.

In Figure 3, the "Remove Undercuts" function is activated. Removing undercuts means that recessed lower portions of teeth (upper portions in the maxilla) are treated as if filled.

FIG. 4 illustrates the state in which the function according to FIG. 3 (removal of undercuts: Block out) has been performed. In FIG. 4, all the undercuts present in each tooth have been removed, that is, the defective portions of the teeth (the white areas in FIG. 4) are shown as being completely filled. The removal of undercuts may be carried out for all the teeth, but may also be performed only for some teeth, as necessary.

In step S051, portions of undercuts having a depth greater than a predetermined value are treated as if no undercut exists (block-out).

Generally, the depth value of the undercut portion of a tooth increases as it moves farther away from the convex region. If an orthodontic appliance is designed by taking into account even very deep undercuts, insertion and removal of the removable orthodontic appliance may become extremely difficult. If the appliance is fabricated in a shape that adheres too closely to the dentition, it may be difficult to wear, and even if worn, it may be difficult to detach the appliance from the teeth.

Due to this issue, it is preferable to design the orthodontic appliance while ignoring the undercut portions that have a depth greater than a predetermined value.

The present invention, while securing orthodontic force, also takes into account the convenience of attaching and detaching the appliance, and proposes that an appropriate depth value at which the undercut can be ignored be set to 0.2 mm to 0.5 mm. In other words, if the depth value of the undercut is about 0.2 mm to 0.5 mm or greater, the undercut beyond such depth is ignored (block-out treatment) when designing the orthodontic appliance. In this case, for the tooth areas having undercuts deeper than the above depth, the appliance is designed so as not to adhere closely.

On the other hand, the above-mentioned appropriate depth value cannot be regarded as an absolute figure, and it may be freely adjusted by the practitioner depending on the specific treatment situation of the patient, such as the shape of each tooth and the magnitude of the orthodontic force, through the menu screen shown in FIG. 3 (it is also possible to set different depth values serving as the block-out criteria for each individual tooth). In some cases, the undercut may be entirely ignored. In such a case, the appropriate depth value becomes 0 (zero).

The orthodontic appliance according to the present invention provides an additional means to facilitate its insertion and removal.

Step S052 is a step of setting the bending angle so that the end portion of the orthodontic appliance is formed to bend in the direction opposite to the tooth, starting from the point where the undercut is considered absent (the point at which the undercut is ignored).

After passing Step S051, when the orthodontic appliance is produced (using a 3D printer), the end portion of the appliance will be formed in a shape that extends straight in the vertical direction. However, for easier wearing of the appliance, it may be preferable for the end portion to be slightly flared outward.

Accordingly, the orthodontic appliance according to the present invention is characterized in that its end portion (the portion from the point where block-out begins, i.e., where the undercut is ignored, to the end) is formed to bend by a predetermined value in the direction opposite to the tooth. In other words, the end portion refers to the part from the starting point of block-out according to the present invention up to the terminal end.

Through repeated testing, the present invention proposes an optimal bending angle of 2° to 4° for the end portion of the orthodontic appliance, which facilitates insertion and removal without causing discomfort during wear. However, depending on the patient's oral environment, the bending angle may be less than 2°, and in some cases, no bending may be formed at all. Since the optimal bending angle of the end portion may vary for each tooth, it is also possible to set different bending angles for different teeth.

Meanwhile, although the end portion of the orthodontic appliance generally extends only to the gum line, under certain special conditions of the teeth (for example, when the crown is very short), the end portion may be designed to extend further. In this case, it is preferable for the gum line to be recessed or removed. For instance, when the crown of a tooth is very short, extending the end portion allows the orthodontic appliance to be worn more stably on the patient's dentition.

Step S09 is a step of restoring the undercut modified through Step S051 to its original shape. In effect, this can be regarded as canceling the block-out.

When the cursor on the screen moves to a specific tooth, the computer recognizes this and restores and stores the undercut of the indicated tooth or the surrounding teeth to its original shape.

If it is determined that manufacturing the orthodontic appliance while recognizing the undercut in its original form will not cause significant problems in wearing or removing the appliance, it is preferable to restore the undercut to its original shape. On the contrary, by restoring the undercut in this way, the inner surface of the orthodontic appliance can be fabricated to closely adhere to the tooth, thereby making it easier to secure orthodontic force on the tooth.

The operation of restoring the undercut can be performed in various ways. This operation may be carried out by moving the cursor tooth by tooth, or by dragging the cursor to designate a certain area (designating multiple teeth at once), in which case all the undercuts within the designated area are restored to their original shape. All such variations are considered to fall within the technical scope of the present invention.

When a component is described as being "connected," "coupled," or "linked" to another component, it should be understood that the component may be directly connected or linked to the other component, but that another component may also be "connected," "coupled," or "linked" between the respective components. Unless otherwise specifically mentioned, the singular form shall also include the plural form.

The above description regarding the configuration and effects is directed to one embodiment of the present invention and is not intended to limit the scope of the claims of the present invention. Various changes and modifications can be made without departing from the technical spirit of the present invention, and it will be apparent to those skilled in the art that such simple design modifications fall within the technical scope of the present invention.

## Claims

1. A method for manufacturing an orthodontic appliance using an insertion path, comprising:
displaying, on a screen, the insertion path for determining the insertion direction of the appliance (S01);
designating a tooth targeted for the insertion path setting (S02);
setting the insertion path for the designated tooth in an arbitrary direction (S03);
displaying, on the screen, undercuts of each tooth caused by the set insertion path (S04);
calculating and storing depth values of the undercuts for each tooth (S05);
calculating and storing the total sum of undercut volumes for each tooth (S06);
setting the insertion path in a different direction and repeating steps S04 through S06 (S07); and
selecting and storing the insertion path having the minimum total undercut volume obtained from step S07 (S08).

2. The method of claim 1, wherein step S02 includes dividing teeth into a first group and a second group (S021), and step S03 includes setting insertion paths for each of the first and second groups designated in step S021 (S031).

3. The method of claim 1 or 2, further comprising step S051, wherein, for each tooth undercut, a portion having a depth greater than a predetermined value is set as having no undercut.
